# EUROPEAN PATENT APPLICATION

(11) **EP 4 289 821 A1**
(43) Date of publication of application: **13.12.2023**
(21) Application number: 22749545.4
(22) Date of filing: 25.01.2022
(51) Int. Cl.: C07D 233/58, C07C 22/08, C07C 25/10, C07C 47/544, C07C 49/786, C07C 69/80, C07C 205/06, C07C 205/44, C07D 209/48, C07D 403/06, C08G 59/40

(54) **CHARGE TRANSFER COMPLEX**

(30) Priority: 03.02.2021 JP 2021016045
(71) Applicant: Adeka Corporation, Tokyo 116-8554 (JP)
(72) Inventor: ENDO, Takeshi, Kitakyushu-shi, Fukuoka 804-0015 (JP); MORI, Yasuyuki, Kitakyushu-shi, Fukuoka 804-0015 (JP); OKANO, Ippei, Kuki-shi, Saitama 346-0101 (JP); OGAWA, Ryo, Kuki-shi, Saitama 346-0101 (JP); UEYAMA, Junji, Kuki-shi, Saitama 346-0101 (JP)
(74) Representative: Forstmeyer, Dietmar
(86) International application number: PCT/JP2022/002630
(87) International publication number: WO 2022/168670

(57) **Abstract**

An objective of the invention is to provide a charge transfer complex capable of obtaining a curable resin composition having an excellent balance between curability and storage stability when used as an epoxy-resin curing agent. The invention is a charge transfer complex having an imidazole moiety as an electron donor moiety. The charge transfer complex may be an assembly wherein electrons included in a compound (a) having an imidazole moiety are accepted by a compound (b) having an electron acceptor moiety, or may be a compound having an imidazole moiety and an electron acceptor moiety in its molecule, and the electron acceptor moiety accepts electrons included in the imidazole moiety.

## Description

### Technical Field

The present invention relates to a charge transfer complex, and more specifically, relates to a charge transfer complex having an imidazole moiety, an epoxy-resin curing agent constituted by the charge transfer complex, and a curable resin composition containing the charge transfer complex.

### Background Art

Epoxy resins have a wide variety of industrial uses as components for coating materials, adhesives, various molding materials, etc.

When employed for such uses, epoxy resin is typically used in combination with a curing agent. Various curing agents are known in the art, with examples including acid anhydride-based curing agents, amine-based curing agents, phenol-based curing agents, etc.

Different curing agents are used for different uses. For example, an imidazole-based curing agent, which is different from an addition polymerization-type curing agent, is an anionic polymerization-type curing agent and can therefore achieve curing even with a small additive amount, and is also useful in terms that it has low volatility and low toxicity and can therefore be suitably used for electric/electronic components.

Using an epoxy-resin curing agent alone, however, poses difficulty in striking a balance between curability and storage stability. To improve storage stability, for example, Patent Literature 1 proposes the use, in an epoxy resin system, of a reaction product between an imidazole compound and an epoxy resin. Patent Literature 2 proposes an epoxy-resin curing agent composition containing a modified imidazole, a modified amine, and a phenol compound.

Unfortunately, performing such modifications causes an increase in viscosity of the modified products, thus impairing handleability and workability and making the product unsatisfactory.

### Citation List

### Patent Literature

Patent Literature 1: US Patent No. 4066625
Patent Literature 2: JP 2007-297493A

### Non-Patent Literature

Non-Patent Literature 1: Journal of Polymer Science, Part A: Polymer Chemistry, 2016, 54, 2680-2688
Non-Patent Literature 2: Journal of Polymer Science, Part A: Polymer Chemistry, 2018, 56, 471-474
Non-Patent Literature 3: Composites Part B, 17 (2019), 107380
Non-Patent Literature 4: Materials Letters, 234 (2019), 379-383

### Summary of Invention

An objective to be achieved by the present invention is to provide a charge transfer complex capable of obtaining a curable resin composition having an excellent balance between curability and storage stability when used as a curing agent for epoxy resins.

As a result of diligent research, Inventors have found that a charge transfer complex having an imidazole moiety as an electron donor is effective as a curing agent for epoxy resins, and further, a resin composition made by employing the complex in combination with an epoxy resin achieves a curable resin composition having an excellent balance between curability and storage stability, thus arriving at the present invention.

That is, the present invention is a charge transfer complex having an imidazole moiety as an electron donor moiety.

The present invention is also an epoxy-resin curing agent constituted by the aforementioned charge transfer complex.

The present invention is also a curable resin composition containing an epoxy resin and the aforementioned charge transfer complex.

The charge transfer complex of the present invention, when used in combination with an epoxy resin, can provide a curable resin composition having excellent curability and storage stability, and is thus useful as an epoxy-resin curing agent. By using the charge transfer complex of the present invention, it is possible to provide a resin composition suitable for such uses as one-pack curing-type coating materials, adhesives, etc.

### Brief Description of Drawings

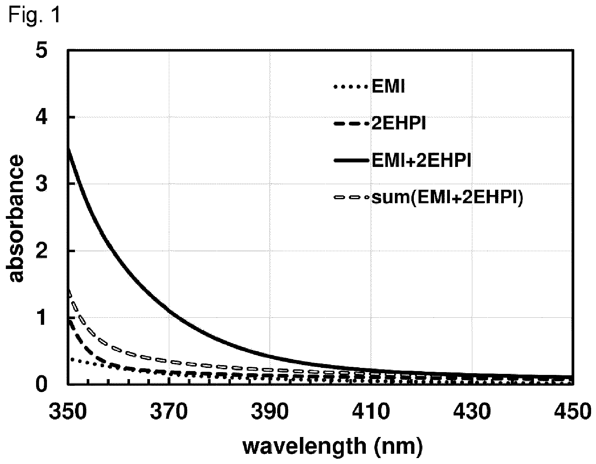

[Fig. 1] Fig. 1 shows UV spectrometric results of a charge transfer complex of the present invention.

### Description of Embodiments

Charge transfer complexes according to the present invention will be described below.

A charge transfer complex, also referred to as an electron-donor-acceptor complex, is an assembly constituted by two or more different molecules wherein electronic charge can be transferred between those molecules, or by a single molecule having, within its molecule, an electron acceptor moiety and an electron donor moiety between which electrons can be transferred.

The charge transfer complex of the present invention is characterized in that the electron donor moiety is an imidazole moiety.

An example of the charge transfer complex may include a charge transfer complex including a compound (a) having an imidazole and a compound (b) having an electron acceptor moiety, wherein an electron included in the compound (a) having an imidazole moiety is accepted by the compound (b) having an electron acceptor moiety. Alternatively, the charge transfer complex may be a compound having an imidazole moiety and an electron acceptor moiety in its molecule, and the electron acceptor moiety accepts an electron included in the imidazole moiety.

Examples of the compound having an imidazole moiety, which is the component (a) of the charge transfer complex, may include: imidazoles having an alkyl group, such as 2-methylimidazole, 1,2-dimethylimidazole, 2-ethyl-4-methylimidazole, 2-isopropylimidazole, 2-undecylimidazole, 2-heptadecylimidazole, etc.; imidazoles having an aromatic group, such as 1-benzyl-2-imidazole, 1-benzyl-2-ethyl-4-methylimidazole, 2-phenylimidazole, 2-phenyl-4-methylimidazole, 1-phenylmethyl-2-phenylimidazole, etc.; imidazoles having an aminoalkyl group, such as 2-aminopropylimidazole, etc.; imidazoles having a cyano group, such as 1-cyanoethyl-2-methylimidazole, 1-cyanoethyl-2-undecylimidazole, 1-cyanoethyl-2-ethyl-4-methylimidazole, 1-cyanoethyl-2-phenylimidazole, etc.; imidazoles having a hydroxymethyl group, such as 2-phenyl-4,5-dihydroxymethylimidazole, 2-phenyl-4-methyl-5-hydroxymethylimidazole, etc.; imidazoles having an ester bond, such as 1-butoxycarbonylethyl-2-methylimidazole, 1-butoxycarbonylethyl-2-ethyl-4-methylimidazole, 1-butoxycarbonylethyl-2-phenylimidazole, 1-(2-ethylhexyl)carbonylethyl-2-methylimidazole, 1-(2-ethylhexyl)carbonylethyl-2-ethyl-4-methylimidazole, 1-(2-ethylhexyl)carbonylethyl-2-phenylimidazole, 1-octyloxycarbonylethyl-2-methylimidazole, 1-octyloxycarbonylethyl-2-ethyl-4-methylimidazole, 1-octyloxycarbonylethyl-2-phenylimidazole, hexanediol·bis(2-methylimidazolyl ethanoic acid) ester, hexanediol·bis(2-ethyl-4-methylimidazolyl ethanoic acid) ester, hexanediol·bis(2-phenylimidazolyl ethanoic acid) ester, decanediol·bis(2-methylimidazolyl ethanoic acid) ester, decanediol·bis(2-ethyl-4-methylimidazolyl ethanoic acid) ester, decanediol bis(2-phenylimidazolyl ethanoic acid) ester, tricyclopentane dimethanol·bis(2-methylimidazolyl ethanoic acid) ester, tricyclopentane dimethanol·bis(2-ethyl-4-methylimidazolyl ethanoic acid) ester, tricyclopentane dimethanol·bis(2-phenylimidazolyl ethanoic acid) ester, 1-(2-hydroxynaphthylmethyl)-2-methylimidazole, 1-(2-hydroxynaphthylmethyl)-2-ethyl-4-methylimidazole, 1-(2-hydroxynaphthylmethyl)-2-phenylimidazole, etc.; special imidazoles, such as 2,3-dihydro-1H-pyrrolo(1,2-a)benzimidazole, 1-dodecyl-2-methyl-3-benzylimidazolium chloride, imidazole silane, 2,4-diamino-6-[2'-methylimidazolyl-(1')]-ethyl-s-triazine, etc. The imidazole compounds given as examples above may be a product modified by, for example, an epoxy compound, a polycarboxylic acid, an isocyanuric acid, etc.

Examples of the epoxy compound may include: polyglycidyl etherified products of mononuclear polyhydric phenol compounds, such as hydroquinone, resorcin, pyrocatechol, phloroglucinol, etc.; polyglycidyl etherified products of polynuclear polyhydric phenol compounds, such as dihydroxynaphthalene, biphenol, methylene bisphenol (bisphenol F), methylene bis(o-cresol), ethylidene bisphenol, isopropylidene bisphenol (bisphenol A), isopropylidene bis(o-cresol), tetrabromobisphenol A, 1,3-bis(4-hydroxycumylbenzene), 1,4-bis(4-hydroxycumylbenzene), 1,1,3-tris(4-hydroxyphenyl)butane, 1, 1,2,2-tetra(4-hydroxyphenyl)ethane, thiobisphenol, sulfobisphenol, oxybisphenol, phenol novolac, o-cresol novolac, ethylphenol novolac, butylphenol novolac, octylphenol novolac, resorcin novolac, terpene phenol, etc.; polyglycidyl etherified products of polyol compounds, such as ethylene glycol, propylene glycol, butylene glycol, hexanediol, polyethylene glycol, polypropylene glycol, thioglycol, dicyclopentadiene dimethanol, 2,2-bis(4-hydroxycyclohexyl)propane (hydrogenated bisphenol A), glycerin, trimethylol propane, pentaerythritol, sorbitol, bisphenol A-alkylene oxide adduct, etc.; glycidyl esterified products of aliphatic, aromatic or alicyclic polybasic acids, such as maleic acid, fumaric acid, itaconic acid, succinic acid, glutaric acid, suberic acid, adipic acid, azelaic acid, sebacic acid, dimer acid, trimer acid, phthalic acid, isophthalic acid, terephthalic acid, trimellitic acid, trimesic acid, pyromellitic acid, tetrahydrophthalic acid, endomethylene tetrahydrophthalic acid, etc.; homopolymers or copolymers of glycidyl methacrylate; epoxy compounds containing a glycidylamino group, such as N,N-diglycidylaniline, bis(4-(N-methyl-N-glycidylamino)phenyl)methane, diglycidyl o-toluidine, N,N-bis(2,3-epoxypropyl)-4-(2,3-epoxypropoxy)-2-methylaniline, N,N-bis(2,3-epoxypropyl)-4-(2,3-epoxypropoxy)aniline, N,N,N',N'-tetra(2,3-epoxypropyl)-4,4-diaminodiphenylmethane, etc.; epoxidized products of cyclic olefin compounds, such as vinylcyclohexene diepoxide, cyclopentadiene diepoxide, 3,4-epoxycyclohexylmethyl-3,4-epoxycyclohexane carboxylate, 3,4-epoxy-6-methylcyclohexylmethyl-6-methylcyclohexane carboxylate, bis(3,4-epoxy-6-methylcyclohexylmethyl)adipate, etc.; epoxidized conjugated diene polymers, such as epoxidized polybutadiene, epoxidized styrene-butadiene copolymer, etc.; and heterocyclic compounds, such as triglycidyl isocyanurate, etc.

Examples of the polycarboxylic acid may include maleic acid, fumaric acid, itaconic acid, succinic acid, glutaric acid, suberic acid, adipic acid, azelaic acid, sebacic acid, dimer acid, trimer acid, phthalic acid, isophthalic acid, terephthalic acid, trimellitic acid, trimesic acid, pyromellitic acid, tetrahydrophthalic acid, endomethylene tetrahydrophthalic acid, etc.

Among the aforementioned imidazole compounds, unmodified compounds, such as 2-methylimidazole, 2-ethyl-4-methylimidazole, etc., are more preferable, because curing is possible with a relatively small amount of addition, and it is also possible to achieve effects as a curing accelerator when used in combination with another curing agent.

As for the compound having an electron acceptor moiety which is the component (b) of the charge transfer complex, it is possible to use, without particular limitation, any compound capable of accepting, in the presence of a compound having an imidazole moiety serving as the component (a), electrons included in the compound having an imidazole moiety as the component (a). Examples may include imide compounds, aromatic compounds, etc.

Among compounds having an electron acceptor moiety, imide compounds are preferable, because there are a wide variety of materials for synthesis and the degree of freedom of molecular design is high, and also, imide compounds have excellent mutual solubility and it is thus possible to easily select a compound that is less likely to negatively affect the physical properties of the obtained curable resin composition. Particularly, imide compounds having an aromatic skeleton in the molecule are preferable, because it is possible to obtain a curable resin composition having an excellent balance between storage stability and curability.

Further, as for the compound having an electron acceptor moiety which is the component (b), it is preferable to employ a compound whose lowest unoccupied molecular orbital (LUMO) is -1 eV or lower, because it is possible to achieve excellent storage stability and curability when such a compound is blended together with an epoxy resin to obtain a curable resin composition. The lowest unoccupied molecular orbital (LUMO) is found, for example, by electronic structure calculation.

The following illustrates concrete examples of compounds having an electron acceptor moiety serving as the component (b).

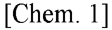

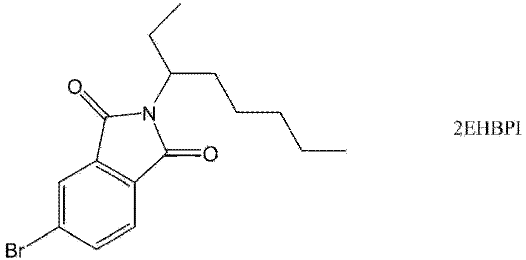

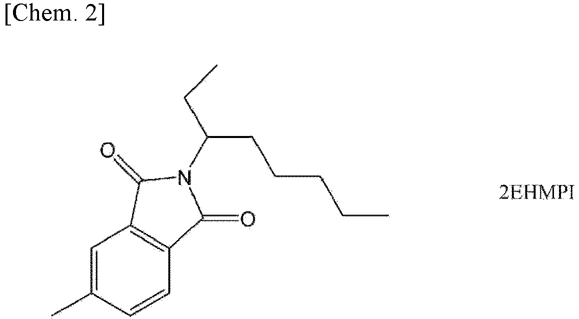

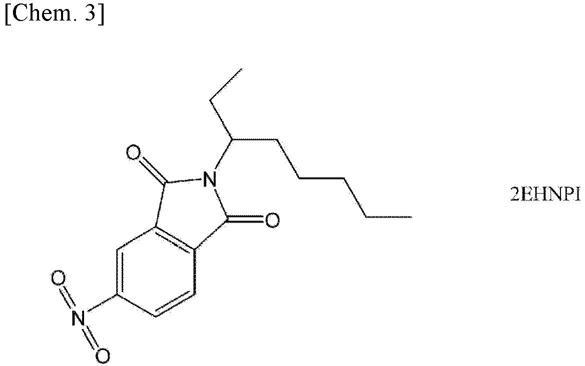

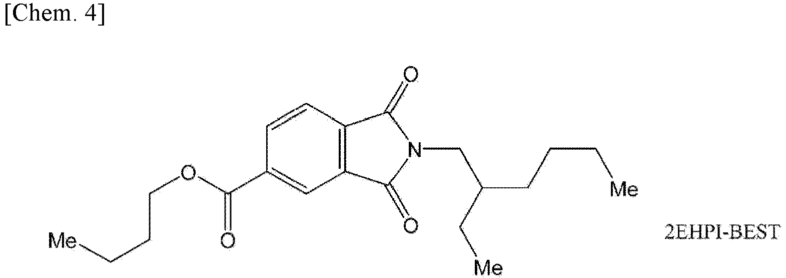

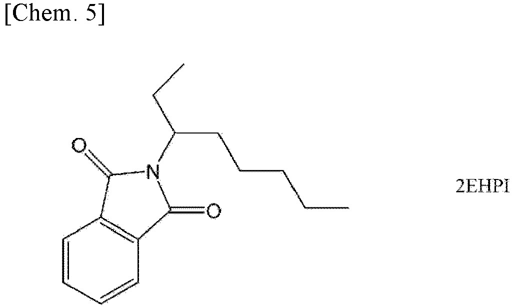

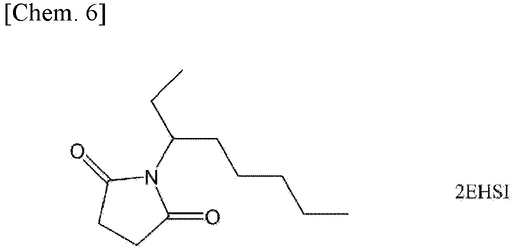

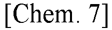

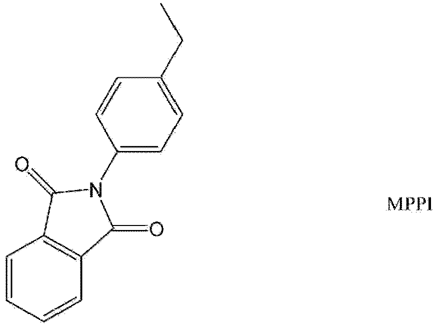

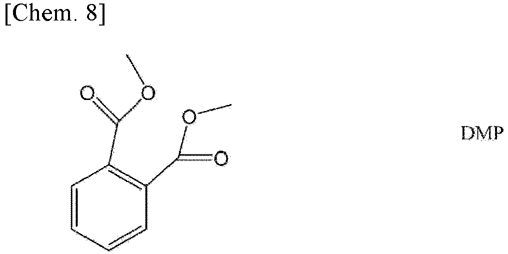

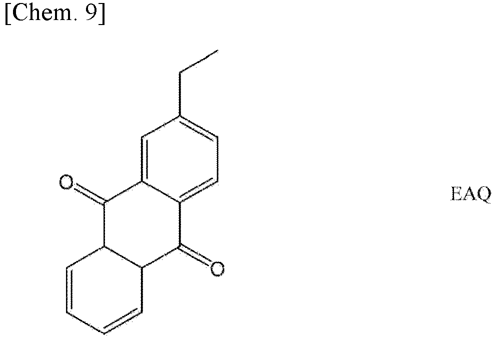

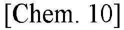

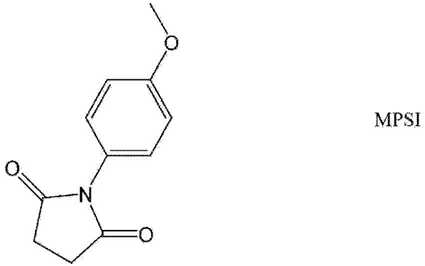

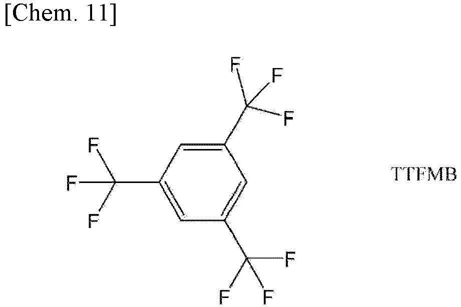

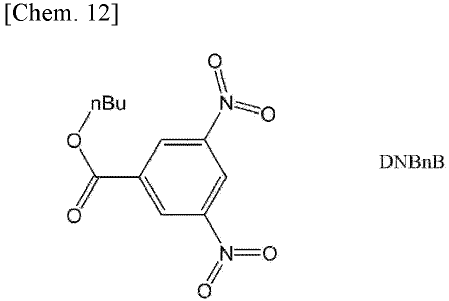

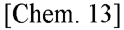

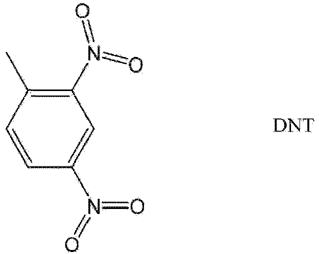

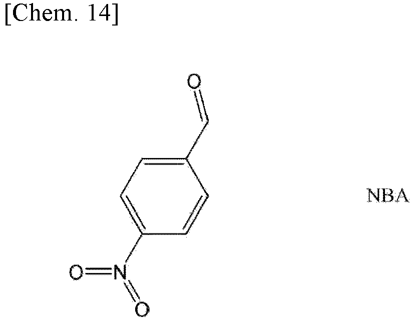

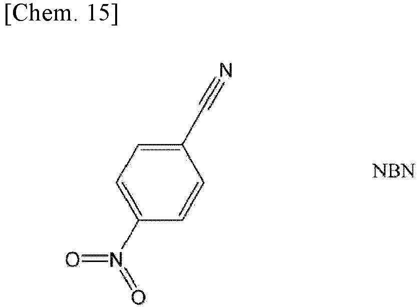

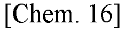

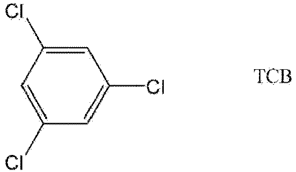

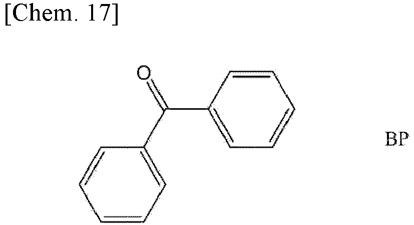

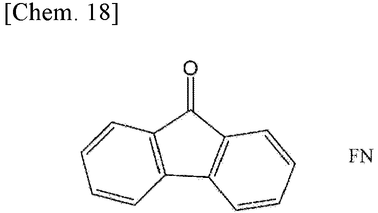

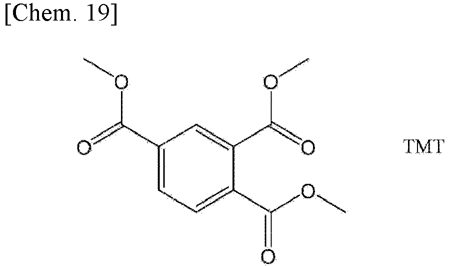

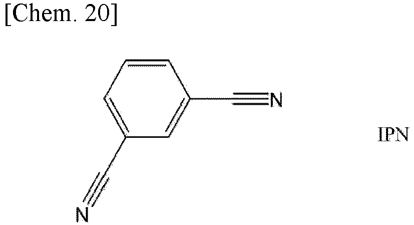

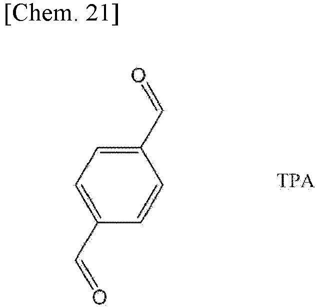

The amount of usage of the compound having an electron acceptor moiety which is the component (b) is preferably from 0.01 to 20 mol, more preferably from 0.1 to 10 mol, even more preferably from 0.2 to 8 mol, with respect to 1 mol of the compound having an imidazole moiety which is the component (a). If the amount of usage of the compound having an electron acceptor moiety is less than 0.01 mol, it may not be possible to achieve the curable resin composition's stability-imparting effect when a charge transfer complex obtained from these compounds is used as an epoxy-resin curing agent. If the amount exceeds 20 mol, the curable resin composition's curability may be negatively affected.

Methods for producing the charge transfer complex are not particularly limited, and the complex can be produced, for example, by mixing the component (a) and the component (b). In cases where both components are in liquid form, a charge transfer complex can be obtained by mixing at atmospheric temperature. In cases where at least one is a solid, the components may be heated and molten and then be mixed, or may be made into a solution using a solvent and then be mixed.

In cases where the charge transfer complex of the present invention is a compound having an imidazole moiety and an electron acceptor moiety in its molecule and the electron acceptor moiety accepts electrons included in the imidazole moiety, it will suffice if the electron acceptor moiety is a moiety that can accept electrons from the imidazole moiety, and examples of the electron acceptor moiety may include an imide moiety, an aromatic moiety, etc. Particularly, it is preferable that the electron acceptor moiety is an imide moiety, because there are a wide variety of materials for synthesis and the degree of freedom of molecular design is high, and also, an imide moiety has excellent mutual solubility and it is thus possible to obtain a compound that is less likely to negatively affect the physical properties of the obtained curable resin composition.

An example of a compound having an imidazole moiety and an electron acceptor moiety in its molecule may include a compound represented by general formula (1) below.

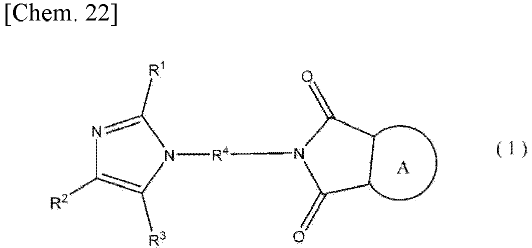

In the general formula (1), R¹, R², and R³ each independently represent a hydrogen atom or a hydrocarbon group having 1 to 20 carbon atoms and optionally having a substituent; R⁴ represents a divalent hydrocarbon group having 1 to 10 carbon atoms and optionally having a substituent; and ring A represents a benzene ring optionally having a substituent, a cyclohexane ring optionally having a substituent, or a norbornene ring optionally having a substituent.

Examples of the hydrocarbon group constituting the hydrocarbon group having 1 to 20 carbon atoms and optionally having a substituent, as represented by R⁶, R⁷, and R⁸ in the formula (1), may include: alkyl groups, such as a methyl group, an ethyl group, a propyl group, an isopropyl group, a butyl group, an isobutyl group, a sec-butyl group, a tert-butyl group, an amyl group, an isoamyl group, a sec-amyl group, a tert-amyl group, a hexyl group, a heptyl group, an octyl group, an isooctyl group, a tert-octyl group, a 2-ethylhexyl group, a nonyl group, an isononyl group, a decyl group, an isodecyl group, an undecyl group, a dodecyl group, a tetradecyl group, a pentadecyl group, a hexadecyl group, a heptadecyl group, an octadecyl group, a nonadecyl group, an eicosyl group, etc.; aryl groups, such as a phenyl group, a naphthyl group, an anthracenyl group, etc.; and alicyclic groups, such as a cyclohexyl group, etc.

Examples of the hydrocarbon group constituting the divalent hydrocarbon group having 1 to 10 carbon atoms and optionally having a substituent, as represented by R⁹, may include a methylene group, an ethylene group, a propylene group, a butylene group, a pentylene group, a hexylene group, a heptylene group, an octylene group, a nonylene group, a decylene group, etc.

Examples of substituents in R⁶ to R⁹ may include alkyl groups, alkoxy groups, haloalkyl groups, haloalkoxy groups, halogen atoms, nitro groups, nitrile groups, amino groups, glycidyl ether groups, etc.

Examples of imidazole skeletons in compounds represented by the general formula (1) may include: imidazole skeletons having an alkyl group, such as 2-methylimidazol-1-yl, 2-ethyl-4-methylimidazol-1-yl, 2-isopropylimidazol-1-yl, 2-undecylimidazol-1-yl, 2-heptadecylimidazol-1-yl, 2-phenylimidazol-1-yl, 2-phenyl-4-methylimidazol-1-yl, etc.; imidazole skeletons having an aromatic group, such as 2-benzylimidazol-1-yl, 2-benzyl-4-methylimidazol-1-yl, 2-phenylmethyl-4-phenylimidazol-1-yl, etc.; imidazole skeletons having an aminoalkyl group, such as 2-aminopropylimidazol-1-yl, etc.; imidazole skeletons having a cyano group, such as 2-cyanoethyl-4-methylimidazol-1-yl, 2-cyanoethyl-4-undecylimidazol-1-yl, 2-cyanoethylmethylimidazol-1-yl, 2-cyanoethyl-4-phenylimidazol-1-yl, etc.; imidazole skeletons having a hydroxymethyl group, such as 2-phenyl-4,5-dihydroxymethylimidazol-1-yl, 2-phenyl-4-methyl-5-hydroxymethylimidazol-1-yl, etc.; and imidazole skeletons having an ester bond, such as 2-butoxycarbonylethyl-4-methylimidazol-1-yl, 2-butoxycarbonylethyl-4-methylimidazol-1-yl, 2-butoxycarbonylethyl-4-phenylimidazol-1-yl, 2-(2-ethylhexyl)carbonylethyl-4-methylimidazol-1-yl, 2-(2-ethylhexyl)carbonylethyl-4-methylimidazol-1-yl, 2-(2-ethylhexyl)carbonylethyl-4-phenylimidazol-1-yl, 2-octyloxycarbonylethyl-4-methylimidazol-1-yl, 2-octyloxycarbonylethyl-4-methylimidazol-1-yl, 2-octyloxycarbonylethyl-4-phenylimidazol-1-yl, etc.

Further, as for the compound represented by the general formula (1), it is preferable to employ a compound having a lowest unoccupied molecular orbital (LUMO) of -1 eV or lower, because it is possible to achieve excellent storage stability and curability when such a compound is blended together with an epoxy resin to obtain a curable resin composition. The lowest unoccupied molecular orbital (LUMO) is found, for example, by electronic structure calculation.

The following illustrates concrete examples of compounds having an imidazole moiety and an electron acceptor moiety in its molecule.

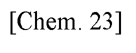

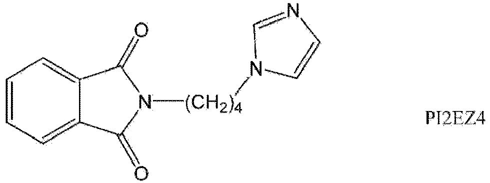

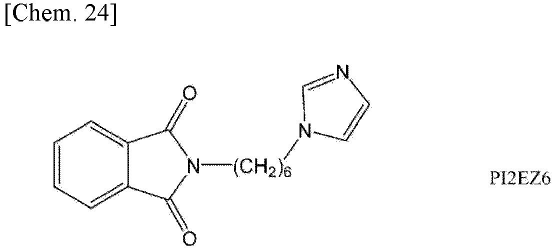

A charge transfer complex is an assembly wherein an electron donor moiety, which donates electrons, and an electron acceptor moiety, which accepts electrons, form a complex. The charge transfer complex causes charge transfer transition in an excited state. From this phenomenon, formation of a charge transfer complex by an electron donor moiety and an electron acceptor moiety can be verified by observing that the UV-vis absorption spectrum after formation of the complex includes a new absorption band on the long-wavelength side of the absorption bands in the UV-vis spectra of the respective materials before formation of the complex.

An epoxy-resin curing agent of the present invention is constituted by the aforementioned charge transfer complex. The charge transfer complex of the present invention is stable in the presence of epoxy resins, and is an excellent curing agent for epoxy resins.

Next, curable resin compositions according to the present invention will be described.

A curable resin composition according to the present invention contains an epoxy resin and the aforementioned charge transfer complex.

The epoxy resin usable in the present invention is not particularly limited in terms of molecular structure, molecular weight, etc., so long as it includes at least two epoxy groups in the molecule.

Examples of the epoxy resin may include: polyglycidyl etherified products of mononuclear polyhydric phenol compounds, such as hydroquinone, resorcin, pyrocatechol, phloroglucinol, etc.; polyglycidyl etherified products of polynuclear polyhydric phenol compounds, such as dihydroxynaphthalene, biphenol, methylene bisphenol (bisphenol F), methylene bis(o-cresol), ethylidene bisphenol, isopropylidene bisphenol (bisphenol A), isopropylidene bis(o-cresol), tetrabromobisphenol A, 1,3-bis(4-hydroxycumylbenzene), 1,4-bis(4-hydroxycumylbenzene), 1,1,3-tris(4-hydroxyphenyl)butane, 1,1,2,2-tetra(4-hydroxyphenyl)ethane, thiobisphenol, sulfobisphenol, oxybisphenol, phenol novolac, o-cresol novolac, ethylphenol novolac, butylphenol novolac, octylphenol novolac, resorcin novolac, terpene phenol, etc.; polyglycidyl etherified products of polyol compounds, such as ethylene glycol, propylene glycol, butylene glycol, hexanediol, polyethylene glycol, polypropylene glycol, thioglycol, dicyclopentadiene dimethanol, 2,2-bis(4-hydroxycyclohexyl)propane (hydrogenated bisphenol A), glycerin, trimethylol propane, pentaerythritol, sorbitol, bisphenol A-alkylene oxide adduct, etc.; glycidyl esterified products of aliphatic, aromatic or alicyclic polybasic acids, such as maleic acid, fumaric acid, itaconic acid, succinic acid, glutaric acid, suberic acid, adipic acid, azelaic acid, sebacic acid, dimer acid, trimer acid, phthalic acid, isophthalic acid, terephthalic acid, trimellitic acid, trimesic acid, pyromellitic acid, tetrahydrophthalic acid, endomethylene tetrahydrophthalic acid, etc.; homopolymers or copolymers of glycidyl methacrylate; epoxy compounds containing a glycidylamino group, such as N,N-diglycidylaniline, bis(4-(N-methyl-N-glycidylamino)phenyl)methane, diglycidyl o-toluidine, N,N-bis(2,3-epoxypropyl)-4-(2,3-epoxypropoxy)-2-methylaniline, N,N-bis(2,3-epoxypropyl)-4-(2,3-epoxypropoxy)aniline, N,N,N',N'-tetra(2,3-epoxypropyl)-4,4-diaminodiphenylmethane, etc.; epoxidized products of cyclic olefin compounds, such as vinylcyclohexene diepoxide, cyclopentadiene diepoxide, 3,4-epoxycyclohexylmethyl-3,4-epoxycyclohexane carboxylate, 3,4-epoxy-6-methylcyclohexylmethyl-6-methylcyclohexane carboxylate, bis(3,4-epoxy-6-methylcyclohexylmethyl)adipate, etc.; epoxidized conjugated diene polymers, such as epoxidized polybutadiene, epoxidized styrene-butadiene copolymer, etc.; and heterocyclic compounds, such as triglycidyl isocyanurate, etc. These epoxy resins may be internally crosslinked by isocyanate-terminal prepolymers, or highly polymerized by using polyvalent active hydrogen compounds (polyhydric phenol, polyamines, carbonyl group-containing compounds, polyphosphoric esters, etc.). The epoxy resin may be used alone, or two or more types may be used in combination.

The curable resin composition of the present invention contains an epoxy resin and the aforementioned charge transfer complex. In cases of using a charge transfer complex obtained from a compound having an imidazole moiety, which is the component (a), and a compound having an electron acceptor moiety, which is the component (b), the curable resin composition can be produced by mixing the charge transfer complex with an epoxy resin. Alternatively, the intended curable resin composition can be produced by separately blending a compound having an imidazole moiety, which is the component (a), and a compound having an electron acceptor moiety, which is the component (b), to an epoxy resin, and forming the charge transfer complex within the curable resin composition.

The amount of the charge transfer complex to be used in the curable resin composition of the present invention is not particularly limited, but is preferably from 0.01 to 500 parts by mass, more preferably from 0.1 to 100 parts by mass, with respect to 100 parts by mass of the epoxy resin. If the content is less than 0.01 parts by mass, it may not be possible to obtain the effect of improving curability and stability. If the amount of use exceeds 500 parts by mass, the physical properties of a cured product may be negatively affected.

Further, an ordinary epoxy-resin curing agent may be used in the curable resin composition of the present invention, in addition to the aforementioned charge transfer complex. Examples of the epoxy-resin curing agent may include acid anhydride-based curing agents, phenol-based curing agents, amine-based curing agents, polythiol-based curing agents, etc.

Examples of the acid anhydride-based curing agents may include hymic anhydride, phthalic anhydride, maleic anhydride, methylhymic anhydride, succinic anhydride, tetrahydrophthalic anhydride, hexahydrophthalic anhydride, methyltetrahydrophthalic anhydride, methylhexahydrophthalic anhydride, trialkyltetrahydrophthalic anhydride-maleic anhydride adducts, benzophenonetetracarboxylic anhydride, trimellitic anhydride, pyromellitic anhydride, hydrogenated methylnadic anhydride, etc.

Examples of the phenol-based curing agents may include polyhydric phenol compounds, such as phenol novolac resin, cresol novolac resin, aromatic hydrocarbon formaldehyde resin-modified phenolic resin, dicyclopentadiene-phenol addition-type resin, phenol aralkyl resin (Xylok resin), naphthol aralkyl resin, trisphenylol methane resin, tetraphenylol ethane resin, naphthol novolac resin, naphthol-phenol co-condensed novolac resin, naphthol-cresol co-condensed novolac resin, biphenyl-modified phenolic resin (a polyhydric phenol compound wherein phenol nuclei are linked by a bismethylene group), biphenyl-modified naphthol resin (a polyhydric naphthol compound wherein phenol nuclei are linked by a bismethylene group), aminotriazine-modified phenolic resin (a compound containing a phenol skeleton, a triazine ring and a primary amino group in its molecular structure), and alkoxy group-containing aromatic ring-modified novolac resin (a polyhydric phenol compound wherein a phenol nucleus and an alkoxy group-containing aromatic ring are linked by formaldehyde).

Examples of the amine-based curing agents may include: alkylene diamines, such as ethylenediamine, 1,2-diaminopropane, 1,3-diaminopropane, 1,3-diaminobutane, 1,4-diaminobutane, hexamethylenediamine, meta-xylenediamine, etc.; polyalkylpolyamines, such as diethylenetriamine, triethylenetriamine, tetraethylenepentamine, etc.; alicyclic polyamines, such as 1,4-diaminocyclohexane, 1,3-diaminocyclohexane, 1,3-diaminomethylcyclohexane, 1,2-diaminocyclohexane, 1,4-diamino-3,6-diethylcyclohexane, 4,4'-diaminodicyclohexylmethane, 1,3-bis(aminomethyl)cyclohexane, 1,4-bis(aminomethyl)cyclohexane, 4,4'-diaminodicyclohexylpropane, bis(4-aminocyclohexyl)sulfone, 4,4'-diaminodicyclohexyl ether, 2,2'-dimethyl-4,4'-diaminodicyclohexylmethane, isophorone diamine, norbornene diamine, etc.; aromatic polyamines, such as diaminodiphenylmethane, diaminodiphenylsulfone, diethyltoluenediamine, 1-methyl-3,5-diethyl-2,4-diaminobenzene, 1-methyl-3,5-diethyl-2,6-diaminobenzene, 1,3,5-triethyl-2,6-diaminobenzene, 3,3'-diethyl-4,4'-diaminodiphenylmethane, 3,5,3',5'-tetramethyl-4,4'-diaminodiphenylmethane, etc.; N,N-dimethylaminoethylamine, N,N-diethylaminoethylamine, N,N-diisopropylaminoethylamine, N,N-diallylaminoethylamine, N,N-benzylmethylaminoethylamine, N,N-dibenzylaminoethylamine, N,N-cyclohexylmethylaminoethylamine, N,N-dicyclohexylaminoethylamine, N-(2-aminoethyl)pyrrolidine, N-(2-aminoethyl)piperidine, N-(2-aminoethyl)morpholine, N-(2-aminoethyl)piperazine, N-(2-aminoethyl)-N'-methylpiperazine, N,N-dimethylaminopropylamine, N,N-diethylaminopropylamine, N,N-diisopropylaminopropylamine, N,N-diallylaminopropylamine, N,N-benzylmethylaminopropylamine, N,N-dibenzylaminopropylamine, N,N-cyclohexylmethylaminopropylamine, N,N-dicyclohexylaminopropylamine, N-(3-aminopropyl)pyrrolidine, N-(3-aminopropyl)piperidine, N-(3-aminopropyl)morpholine, N-(3-aminopropyl)piperazine, N-(3-aminopropyl)-N'-methylpiperidine, 4-(N,N-dimethylamino)benzylamine, 4-(N,N-diethylamino)benzylamine, 4-(N,N-diisopropylamino)benzylamine, N,N-dimethylisophoronediamine, N,N-dimethylbisaminocyclohexane, N,N,N'-trimethylethylenediamine, N'-ethyl-N,N-dimethylethylenediamine, N,N,N'-triethylethylenediamine, N'-ethyl-N,N-dimethylpropanediamine, N'-ethyl-N,N-dibenzylaminopropylamine; N,N-(bisaminopropyl)-N-methylamine, N,N-bisaminopropylethylamine, N,N-bisaminopropylpropylamine, N,N-bisaminopropylbutylamine, N,N-bisaminopropylpentylamine, N,N-bisaminopropylhexylamine, N,N-bisaminopropyl-2-ethylhexylamine, N,N-bisaminopropylcyclohexylamine, N,N-bisaminopropylbenzylamine, N,N-bisaminopropylallylamine, bis[3-(N,N-dimethylaminopropyl)]amine, bis[3-(N,N-diethylaminopropyl)]amine, bis[3-(N,N-diisopropylaminopropyl)]amine, bis[3-(N,N-dibutylaminopropyl)]amine; dibasic acid dihydrazides, such as oxalic acid dihydrazide, malonic acid dihydrazide, succinic acid dihydrazide, glutaric acid dihydrazide, adipic acid dihydrazide, suberic acid dihydrazide, azelaic acid dihydrazide, sebacic acid dihydrazide, phthalic acid dihydrazide, etc.; guanidine compounds, such as dicyandiamide, benzoguanamine, acetoguanamine, etc.; and melamine, etc.

It is also possible to use modified amine-based curing agents obtained by modifying the aforementioned amines. Examples of modification methods may include dehydration condensation with carboxylic acids, addition reaction with epoxy resins, addition reaction with isocyanates, Michael addition reaction, Mannich reaction, condensation reaction with urea, and condensation reaction with ketones.

Examples of carboxylic acids that can be used for modification of the aforementioned amines may include aliphatic, aromatic or alicyclic polybasic acids such as maleic acid, fumaric acid, itaconic acid, succinic acid, glutaric acid, suberic acid, adipic acid, azelaic acid, sebacic acid, dimer acid, trimer acid, phthalic acid, isophthalic acid, terephthalic acid, trimellitic acid, trimesic acid, pyromellitic acid, tetrahydrophthalic acid, hexahydrophthalic acid, endomethylene tetrahydrophthalic acid, etc.

Examples of epoxy compounds that can be used for modification of the aforementioned amines may include epoxy compounds given as examples of the epoxy compounds used for modification of the compound having an imidazole moiety, which is the component (a) of the charge transfer complex.

Examples of isocyanate compounds that can be used for modification of the aforementioned amines may include: aromatic diisocyanates, such as 2,4-tolylene diisocyanate, 2,6-tolylene diisocyanate, diphenylmethane-4,4'-diisocyanate, phenylene diisocyanate, xylylene diisocyanate, tetramethylxylylene diisocyanate, 1,5-naphthylene diisocyanate, 1,5-tetrahydronaphthalene diisocyanate, 3,3'-dimethyldiphenyl-4,4'-diisocyanate, dianisidine diisocyanate, tetramethylxylylene diisocyanate, etc.; alicyclic diisocyanates, such as isophorone diisocyanate, dicyclohexylmethane-4,4'-diisocyanate, trans-1,4-cyclohexyl diisocyanate, norbornene diisocyanate, etc.; aliphatic diisocyanates, such as tetramethylene diisocyanate, 1,6-hexamethylene diisocyanate, 2,2,4- and/or 2,4,4-trimethylhexamethylene diisocyanate, lycine diisocyanate, etc.; isocyanurate trimers, biuret trimers, trimethylolpropane adducts, etc., of the aforementioned diisocyanates; triphenylmethane triisocyanate, 1-methylbenzol-2,4,6-triisocyanate, dimethyltriphenylmethane tetraisocyanate, etc. Further, the aforementioned isocyanate compounds may take the form of a modified product, such as carbodiimide-modified, isocyanurate-modified, biuret-modified, etc., or may take the form of blocked isocyanate which is blocked by a variety of blocking agents.

Examples of the polythiol-based curing agents may include pentaerythritol tetrakis(3-mercaptopropionate), pentaerythritol tetrakis(thioglycolate), dipentaerythritol hexakis(3-mercaptopropionate), dipentaerythritol hexakis(3-mercaptobutyrate), 1,3,4,6-tetrakis(2-mercaptoethyl)-1,3,4,6-tetraazaoctahydropentalene-2,5-dione, 1,3,5-tris(3-mercaptopropyl)-1,3,5-triazine-2,4,6(1H,3H,SH)-trione, 4-mercaptomethyl-1,8-dimercapto-3,6-dithiaoctane, 4,8-, 4,7- or 5,7-dimercaptomethyl-1,11-dimercapto-3,6,9-trithiaundecane, 1,3,4,6-tetrakis(2-mercaptoethyl)glycoluril, etc.

Examples of the imidazole-based curing agents may include 2-methylimidazole, 2-ethyl-4-methylimidazole, 2-isopropylimidazole, 2-undecylimidazole, 2-heptadecylimidazole, 2-phenylimidazole, 2-phenyl-4-methylimidazole, 2-aminopropylimidazole, imidazole silane (for example, 2MUSIZ from Shikoku Chemicals Corporation), etc.

It is also possible to use modified products obtained by modifying these imidazole compounds in the same manner as the aforementioned modified amine-based curing agents, or imidazole salts between the aforementioned imidazoles and trimellitic acid, isocyanuric acid, boron, etc.

Examples of commercially available curing agents may include: Adeka Hardener EH-3636AS and Adeka Hardener EH-4351S (from Adeka Corporation; dicyandiamide-type latent curing agent); Adeka Hardener EH-5011S and Adeka Hardener EH-5046S (from Adeka Corporation; imidazole-type latent curing agent); Adeka Hardener EH-4357S, Adeka Hardener EH-5057P, and Adeka Hardener EH-5057PK (from Adeka Corporation; polyamine-type latent curing agent); Ajicure PN-23 and Ajicure PN-40 (from Ajinomoto Fine-Techno Co., Inc.; amine adduct-based latent curing agent); Ajicure VDH (from Ajinomoto Fine-Techno Co., Inc.; hydrazide-based latent curing agent); Fujicure FXR-1020 (from T&K TOKA Corporation; latent curing agent); Curezol (from Shikoku Chemicals Corporation; imidazole-based curing agent); TS-G (from Shikoku Chemicals Corporation; polythiol-based curing agent); DPMP and PEMP (from SC Organic Chemical Co., Ltd.; polythiol-based curing agent); and PETG (from Yodo Kagaku Co., Ltd.; polythiol-based curing agent), etc.

The curing agent may be used alone, or two or more types may be used in combination.

The amount of curing agent to be blended is not particularly limited, but is preferably from 0 to 500 parts by mass, more preferably from 0 to 100 parts by mass, with respect to 100 parts by mass of the epoxy resin.

In the present invention, it is possible to use, in combination, the aforementioned curing agent and a known epoxy-resin curing accelerator, as necessary. Examples of the curing accelerator may include: phosphines, such as triphenylphosphine, etc.; phosphonium salts, such as tetraphenylphosphonium bromide, etc.; amines, such as benzyldimethylamine, 2,4,6-tris(dimethylaminomethyl)phenol, etc.; quaternary ammonium salts, such as trimethylammonium chloride, etc.; ureas, such as 3-(p-chlorophenyl)-1,1-dimethylurea, 3-(3,4-dichlorophenyl)-1,1-dimethylurea, 3-phenyl-1,1-dimethylurea, isophorone diisocyanate-dimethylurea, tolylene diisocyanate-dimethylurea, etc.; and complexes between boron trifluoride and amines, complexes between boron trifluoride and ether compounds, etc. The curing accelerator may be used alone, or two or more types may be used in combination. The content of the epoxy-resin curing accelerator is not particularly limited, and may be set as appropriate depending on the use of the curable resin composition.

The curable resin composition of the present invention may contain a silane coupling agent. Examples of the silane coupling agent may include γ-aminopropyltriethoxysilane, N-β-(aminoethyl)-γ-aminopropyltriethoxysilane, N-β-(aminoethyl)-N'-β-(aminoethyl)-γ-aminopropyltriethoxysilane, γ-anilinopropyltriethoxysilane, γ-glycidoxypropyltriethoxy silane, β-(3,4-epoxycyclohexyl)ethyltriethoxysilane, vinyltriethoxysilane, N-β-(N-vinylbenzylaminoethyl)-γ-aminopropyltriethoxysilane, γ-methacryloxypropyltrimethoxysilane, γ-chloropropyltrimethoxysilane, γ-mercaptopropyltrimethoxysilane, etc.

The curable resin composition of the present invention may contain a filler. Examples of the filler may include: silica, such as fused silica, crystalline silica, etc.; powders, such as magnesium hydroxide, aluminum hydroxide, zinc molybdate, calcium carbonate, silicon carbonate, calcium silicate, potassium titanate, beryllia, zirconia, zircon, forsterite, steatite, spinel, mullite, titania, etc., and beads obtained by conglobing the above; and fibers, such as glass fiber, pulp fiber, synthetic fiber, ceramic fiber, etc.

The curable resin composition of the present invention may be used by being dissolved in one or more of various solvents, preferably organic solvents. Examples of suitable organic solvents may include: ethers, such as tetrahydrofuran, 1,2-dimethoxyethane, 1,2-diethoxyethane, etc.; alcohols, such as iso- or n-butanol, iso- or n-propanol, amyl alcohol, benzyl alcohol, furfuryl alcohol, tetrahydrofurfuryl alcohol, etc.; ketones, such as methyl ethyl ketone, methyl isopropyl ketone, methyl butyl ketone, etc.; aromatic hydrocarbons, such as benzene, toluene, xylene, etc.; triethylamine, pyridine, dioxane, acetonitrile, etc.

The curable resin composition of the present invention may further contain other types of additives, as necessary. Examples of the additives may include: phosphorus-based antioxidants, phenol-based antioxidant, sulfur-based antioxidants, etc.; UV absorbers and hindered-amine-based light stabilizers; phenol compounds, such as biphenol, etc.; reactive diluents, such as monoalkyl glycidyl ether, etc.; non-reactive diluents (plasticizers), such as dioctyl phthalate, dibutyl phthalate, benzyl alcohol, coal tar, etc.; reinforcing materials, such as glass cloth, aramid cloth, carbon fiber, etc.; pigments; lubricants, such as candelilla wax, carnauba wax, Japan wax, Chinese wax, beeswax, lanolin, spermaceti wax, montan wax, petroleum wax, aliphatic wax, aliphatic ester, aliphatic ether, aromatic ester, aromatic ether, etc.; thickeners; thixotropic agents; antifoaming agents; rust preventives; and commonly used additives, such as colloidal silica, colloidal alumina, etc. In the present invention, an adhesive resin, such as cyanate ester resin, xylene resin, petroleum resin, etc., may be used in combination.

The uses of the resin composition of the present invention are not particularly limited, but since the resin composition allows the balance between curability and storage stability to be adjusted, it can therefore be used as a one-pack curing-type resin composition, and can be used, for example, as coating materials and adhesives for concrete, cement mortar, various metals, leather, glass, rubber, plastic, wood, cloth, paper, etc.

### Examples

Next, the present invention will be described in further detail below according to Examples and Comparative Example. The invention, however, is not to be limited to the following Examples.

### {Calculation of Lowest Unoccupied Molecular Orbital (LUMO)}

The lowest unoccupied molecular orbital (LUMO) of each compound having an electron acceptor moiety, as well as that of each compound having an imidazole moiety and an electron acceptor moiety in its molecule, used in the respective Examples was calculated using Gaussian 09, EM64L-G09 Rev. B.01; structural optimization was performed without symmetry constraints by using density functional theory at the B3LYP/6-31G (d, p) level, and calculation was carried out after confirming, for each optimized structure, that the stationary point was a local minimum through harmonic vibrational frequency calculation. The results are shown in Table 1.

### {Example 1}

2-Ethyl-4-methylimidazole (EMI) and N-(2-ethylhexyl)phthalimide (2EHPI) were mixed at a ratio of 1:1, to obtain a uniform mixture. The measurement result of the UV spectrum of the obtained mixture (EMI+2EHPI) is shown in Fig. 1.

For comparison, the UV spectrum of 2-ethyl-4-methylimidazole (EMI) and the UV spectrum of N-(2-ethylhexyl)phthalimide (2EHPI) were measured. These UV spectra and the combined UV spectrum which is the sum thereof ("sum(EMI+2EHPI)") are also shown in Fig. 1.

These results show that the UV spectrum of the uniform mixture obtained by mixing 2-ethyl-4-methylimidazole (EMI) and N-(2-ethylhexyl)phthalimide (2EHPI) at a ratio of 1:1 was shifted toward the long-wavelength side compared to the combined UV spectrum of the sum of EMI and 2EHPI, showing that a charge transfer complex was formed.

### {Examples 2 to 18 and Comparative Example 1}

Bisphenol A-type epoxy resin (BISAEP), 2-ethyl-4-methylimidazole (EMI), and respective compounds given as concrete examples of compounds having an electron acceptor moiety were blended according to the blending ratio (mol) shown in Table 1, to produce respective curable resin compositions containing a charge transfer complex. The obtained curable resin compositions were used to conduct the evaluations described below.

### {Examples 19 and 20}

Bisphenol A-type epoxy resin (BISAEP) and respective compounds given as concrete examples of compounds having an imidazole moiety and an electron acceptor moiety in its molecule were blended according to the blending ratio (mol) shown in Table 1, to produce respective curable resin compositions. The obtained curable resin compositions were used to conduct the evaluations described below.

### {Curability}

Each curable resin composition was placed in a glass vial and heated at 150°C for 1 hour to cure. Compositions that had no tack and that cured to a completely solid state were evaluated as ∘ (white circle), whereas compositions other than the above were evaluated as × (cross).

### {Storage Stability}

Each curable resin composition was charged into a 13-mm-dia. 40-mm high glass vial so as to fill one-fifth of the vial from the bottom, and the vial was covered with a cap. The vial was tilted by 90° on a flat desk, and the state after 1 minute was observed to see whether the composition had flowability. Cases in which a change was observed in the form of the curable resin composition were considered as having flowability; whereas cases where no change was observed were considered as having no flowability. This evaluation was carried out every time the sample was left to stand for 1 day, and the evaluation was terminated when flowability was no longer observed. Table 1 shows the number of days that flowability could be maintained. Cases in which flowability could be maintained for 3 or more days were evaluated as "pass" (∘; white circle) in terms of storage stability, whereas cases that did not satisfy this condition were evaluated as "fail" (×; cross) in terms of storage stability.

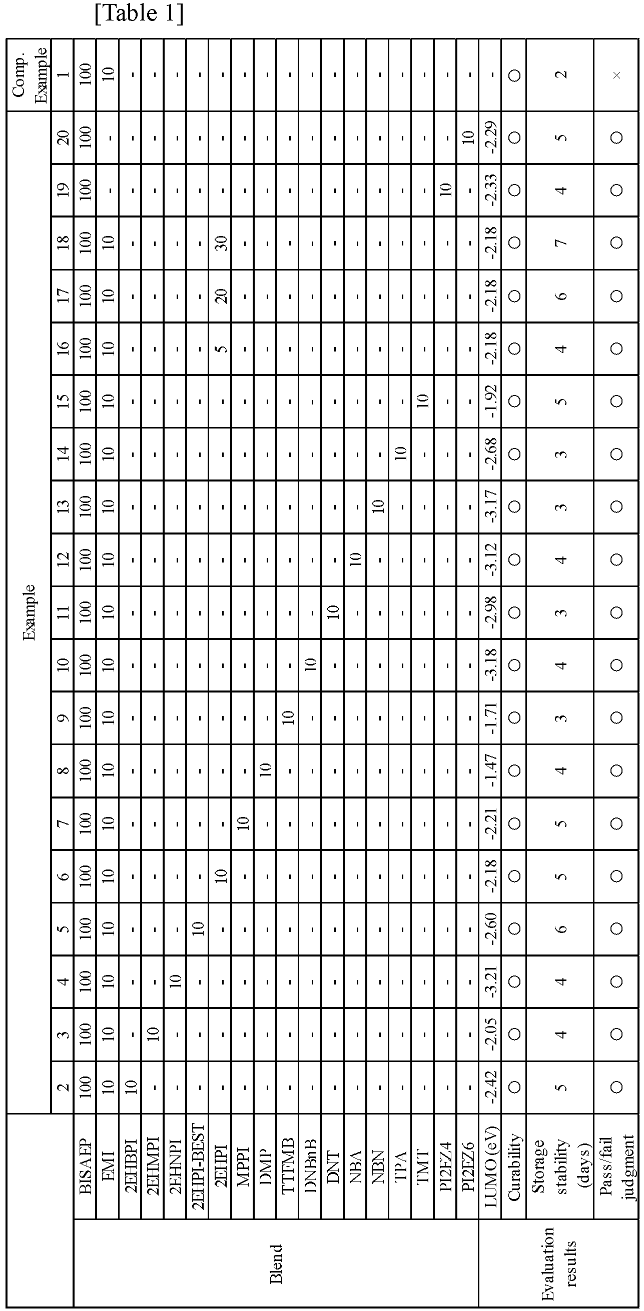

The Examples show that novel charge transfer complexes were obtained. The results also clearly show that these charge transfer complexes are useful as epoxy-resin curing agents, and curable resin compositions obtained by including an epoxy resin and the respective charge transfer complex have excellent curability and storage stability.

### Industrial Applicability

According to the present invention, it is possible to provide a one-pack curing-type curable resin composition that particularly has excellent curability and storage stability, and that can suitably be used, for example, for adhesives for electronic components, sealing materials for electronic components, casting materials, coating materials, structural adhesives, etc.

## Claims

1. A charge transfer complex comprising an imidazole moiety as an electron donor moiety.

2. The charge transfer complex according to claim 1, comprising compound (a) having an imidazole moiety and compound (b) having an electron acceptor moiety,
wherein an electron included in the compound (a) is accepted by the compound (b).

3. The charge transfer complex according to claim 2, wherein the compound (b) having an electron acceptor moiety has a lowest unoccupied molecular orbital (LUMO) of -1 eV or lower.

4. The charge transfer complex according to claim 2 or 3, wherein the compound (b) having an electron acceptor moiety is a compound having an imide moiety.

5. The charge transfer complex according to claim 1, wherein the charge transfer complex is a compound having an imidazole moiety and an electron acceptor moiety in its molecule, and the electron acceptor moiety accepts an electron included in the imidazole moiety.

6. The charge transfer complex according to claim 5, wherein the compound having an imidazole moiety and an electron acceptor moiety in its molecule has a lowest unoccupied molecular orbital (LUMO) of -1 eV or lower.

7. The charge transfer complex according to claim 5 or 6, wherein the electron acceptor moiety is an imide moiety.

8. The charge transfer complex according to any one of claims 5 to 7, wherein the compound having an imidazole moiety and an electron acceptor moiety in its molecule is a compound represented by formula (1) below: 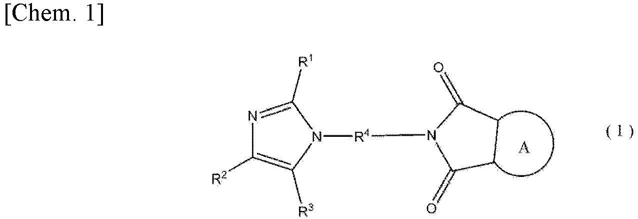
wherein, R¹, R², and R³ each independently represent a hydrogen atom or a hydrocarbon group having 1 to 20 carbon atoms and optionally having a substituent;
R⁴ represents a divalent hydrocarbon group having 1 to 10 carbon atoms and optionally having a substituent; and
ring A represents a benzene ring optionally having a substituent, a cyclohexane ring optionally having a substituent, or a norbornene ring optionally having a substituent.

9. An epoxy-resin curing agent comprising the charge transfer complex according to any one of claims 1 to 8.

10. A curable resin composition comprising an epoxy resin and the charge transfer complex according to any one of claims 1 to 8.
